# EUROPÄISCHE PATENTSCHRIFT

(11) **EP 1 270 586 B1**
(45) Veröffentlichungstag und Bekanntmachung des Hinweises auf die Patenterteilung: **29.11.2006**
(21) Anmeldenummer: 02014449.9
(22) Anmeldetag: 28.06.2002
(51) Int. Cl.: C07K 14/025, C12N 15/37, A61K 47/48, A61K 48/00, C12N 15/88

(54) **Zusammensetzung zum zellspezifischen Transfer von Wirkstoffen**
Composition for the cell-specific transfer of active substances
Composition pour le transfère spécifique des substances actives

(30) Priorität: 28.06.2001 DE 10131145
(43) Veröffentlichungstag der Anmeldung: 02.01.2003
(73) Patentinhaber: Forschungszentrum für Medizintechnik und Biotechnologie E.V., 99947 Bad Langensalza (DE)
(72) Erfinder: Lüke, Wolfgang, Dr., 37083 Göttingen (DE); Petry, Harald, Dr., 37130 Gleichen (DE); Ast, Oliver, Dr., 37127 Dransfeld (DE); Wilke, Ingo, Dr., 37133 Friedland Ballenhausen (DE); Goldmann, Claudia, Dr., 37077 Göttingen (DE); Wagner, Kerstin, Dr., 07745 Jena (DE); Schnabelrauch, Matthias, Dr., 07745 Jena (DE)
(74) Vertreter: Prechtel, Jörg

(56) Entgegenhaltungen:
- US-A- 5 789 230
- US-B1- 6 238 859
- GOLDMANN CLAUDIA ET AL: "Molecular cloning and expression of major structural protein VP1 of the human polyomavirus JC virus: Formation of virus-like particles useful for immunological and therapeutic studies." JOURNAL OF VIROLOGY, Bd. 73, Nr. 5, Mai 1999 (1999-05), Seiten 4465-4469, XP002249834 ISSN: 0022-538X
- GOLDMANN C ET AL: "PACKAGING OF SMALL MOLECULES INTO VP1-VIRUS-LIKE PARTICLES OF THE HUMAN POLYOMAVIRUS JC VIRUS" JOURNAL OF VIROLOGICAL METHODS, AMSTERDAM, NL, Bd. 90, Nr. 1, Oktober 2000 (2000-10), Seiten 85-90, XP001006271 ISSN: 0166-0934
- GOLDMANN C ET AL: "Cell-specific and efficient gene delivery systems based on virus-like particles" JOURNAL OF MOLECULAR MEDICINE, SPRINGER VERLAG, DE, Bd. 7, Nr. 75, 1. Juli 1997 (1997-07-01), Seiten b210-b211, XP002079918 ISSN: 0946-2716
- WICKHAM T J: "TARGETING ADENOVIRUS" GENE THERAPY, MACMILLAN PRESS LTD., BASINGSTOKE, GB, Bd. 7, Nr. 2, Januar 2000 (2000-01), Seiten 110-114, XP001055892 ISSN: 0969-7128

## Beschreibung

Die Erfindung betrifft neue Zusammensetzungen zum zellspezifischen Transfer von Wirkstoffen auf Basis viraler Strukturproteine, die mit einem kationischen Polymer als Anker zur Bindung von Liganden, insbesondere zielzellspezifischen Liganden assoziiert sind.

Die klinische Anwendbarkeit von gentherapeutischen Verfahren ist im Wesentlichen von der Effizienz, der Zielzellspezifität und der biologischen Sicherheit des verwendeten Transfersystems abhängig, mit dem die therapeutische DNA in die Zellen eingebracht wird. Alle bislang verfügbaren Transfersysteme weisen hinsichtlich dieser Eigenschaften wesentliche Probleme auf. Virale Transfersysteme bergen aufgrund der potenziellen Rekombinationsgefahr mit zellulären Sequenzen ein kaum kalkulierbares Sicherheitsrisiko. Adenoviren und adenoassoziierte Viren, die derzeit favorisierten Systeme zum Transport von therapeutischen Genen, machen aufgrund ihrer hohen Immunogenität bei den meisten Patienten eine mehrmalige in vivo Applikation unmöglich. Nicht-virale Systeme, wie Liposomen und DNA-kondensierende Moleküle vermeiden zwar diese Nachteile, zeigen dafür allerdings, ähnlich wie retrovirale Systeme, weitaus geringere Transfereffizienzen und Zielzellspezifitäten.

Um diese Probleme zu umgehen, wurde ein neuartiges Transfersystem auf der Basis von virus-ähnlichen Partikeln (VLP) entwickelt (WO 97/19174). Diese VLP können durch rekombinante Expression des Hauptstrukturproteins VP1 des humanen Polyomavirus JCV in Insektenzellen hergestellt werden. Grundlage dieses Systems ist die Eigenschaft der VP1-VLP DNA verpacken zu können und diese dann spezifisch in bestimmte Zellen einzuschleusen. Im Gegensatz zur VP1-Expression anderer Polyomaviren werden die VP1-VLPs in den Zellkulturüberstand sezerniert, aus dem sie durch zwei aufeinander folgende Zentrifugationsschritte in hoher Reinheit präpariert werden. VP1-VLPs können durch Entzug von Ca²⁺-lonen unter reduzierenden Bedingungen in VP1 Pentamere dissoziiert und im Gegensatz zu VP1-VLPs anderer Polyomaviren anschließend wieder zu vollständigen VP1-VLPs reassoziiert werden. Die Verpackung der DNA erfolgt während dieses VP1 Reassoziationsprozesses unter definierten in vitro Bedingungen ohne das sich hierbei die morphologischen und biologischen Eigenschaften der VP1-VLP verändern. Die so verpackte DNA ist im Weiteren geschützt vor enzymatischen Abbau durch DNase I. Darüber hinaus konnte gezeigt werden, dass sich neben der DNA auch niedermolekulare Substanzen in die VP1-VLPs verpacken lassen. Mit Hilfe der VP1-VLP wird die verpackte Fremd-DNA effizient und spezifisch in Zellen renalen und neuronalen Ursprungs eingeschleust und dort exprimiert. Dieser, im Gegensatz zu anderen Transfersystemen, sehr enge Zelltropismus der VP1-VLP entspricht dem des natürlichen JC-Virus und ist für die Anwendung als DNA Transfersystem sehr vorteilhaft.

Neben der biologischen Sicherheit und der Transfereffizienz ist die Zielzellspezifität von Transfersystemen eines der entscheidenden Kriterien für den in vivo Einsatz zur gentherapeutischen Behandlung von Erkrankungen. Viele der derzeit verfügbaren viralen und nicht-viralen Transfersysteme weisen ein sehr breites Wirtszellspektrum auf. Durch eine gezielte Veränderung oder den Austausch von Hüllproteinen wird hierbei versucht, den Tropismus auf bestimmte Zellen oder Gewebe zu beschränken. Diese Strukturveränderungen sind zum einen sehr aufwendig und gehen darüber hinaus häufig zu Lasten der Transfereffizienz.

Die Eignung der VP1-VLPs als zellspezifisches DNA Transport- und Transduktionssystem wurde in verschiedenen Zelllinien untersucht. Immunfluoreszenzuntersuchungen zeigten, dass die VP1-VLP ausschließlich an Zellen renalen und neuronalen Ursprungs binden und innerhalb kurzer Zeit internalisiert und in den Zellkern transportiert werden.

Ein Gegenstand der Erfindung ist somit die Verwendung von VP1 - VLP zur spezifischen Transduktion von Zellen renalen und neuronalen Ursprungs.

Ein weiterer Gegenstand der hier beschriebenen Erfindung ist eine Methode den Zelltropismus der VP1-VLP gezielt zu verändern, um auf diese Weise selektiv ganz bestimmte Zielzellen und Gewebe zu transduzieren. Dieses Prinzip ermöglicht einen flexiblen Einsatz der VP1-VLP als DNA Transfersystem bei der Behandlung von Erkrankungen, die auf bestimmte Zelltypen bzw. Gewebe beschränkt sind.

Vor diesem Hintergrund wurde überraschenderweise gefunden, dass sich die Zielzellspezifität durch Beladung der VP1-VLP mit kationischen Polymeren als Ankermolekül für zellspezifische Liganden gezielt verändern lässt. Daher können die VP1-VLP als zellspezifisches Transportsystem für therapeutische Nukleinsäuren oder Substanzen eingesetzt werden. Vor dem Hintergrund, dass das VP1 Protein rekombinant und getrennt von der therapeutischen DNA in großen Mengen und hoher Reinheit produziert wird und die DNA Verpackung nicht wie bei retroviralen, adeno-assoziierten oder adenoviralen Vektoren in Verpackungszelllinien erfolgt, lassen sich Kontaminationen mit viralen Nukleinsäuren und die potenzielle Gefahr der Entstehung infektiöser Viren aufgrund von Rekombinationsereignissen ausschließen. Da weiterhin die DNA Verpackung und die Beladung der VP1-VLP mit zellspezifischen Liganden unter definierten in vitro Bedingungen erfolgt, stellt das VP1-VLP DNA Transfersystem eine biologisch sichere Platformtechnologie dar, die die Vorteile viraler und nicht-viraler Systeme vereinigt, ohne allerdings deren Nachteile aufzuweisen.

Ein Gegenstand der Erfindung sind somit Konjugate virusähnlicher Partikel (VLP), die aus mehreren Molekülen des Virusproteins VP1 von JC-Virus aufgebaut sind, assoziiert mit einem kationischen Polymer, das als Anker zur Bindung weiterer Liganden dienen kann. Die erfindurigsgemäßen VLP zeichnen sich insbesondere dadurch aus, dass sie frei von mit JCV assoziierten Nukleinsäuren sind. Derartige VLP, insbesondere VLP aus rekombinanten VP1-Molekülen, sind in WO97/19174 beschrieben.

Das VP1 von JVC ist das Hauptstrukturprotein der Kapsidhülle von JCV, z.B. aus Wildtypstämmen oder mutagenisierten Stämmen von JCV. In einer besonderen Ausführungsform ist das VLP aus rekombinant hergestelltem VP1 aufgebaut. Daher umfasst der Begriff VP1 auch Proteine, die sich vom Wildtyp VP1 durch Mutationen, wie etwa Substitutionen, Insertionen oder/und Deletionen, unterscheiden. Zur Herstellung von rekombinanten VP1 verwendet man bevorzugt eine Nukleinsäure, welche die in SEQ. ID NO. 1 gezeigte oder eine dazu komplementäre Sequenz, eine dieser Sequenz im Rahmen der Degeneration des genetischen Codes entsprechende Sequenz oder eine damit unter stringenten Bedingungen hybridisierende Sequenz umfasst, wobei man die Nukleinsäuresequenz oder einen diese Sequenz enthaltenden rekombinanten Vektor in eine geeignete Wirtszelle einbringt, die Wirtszelle unter Bedingungen kultiviert, bei denen eine Expression der Nukleinsäuresequenz erfolgt und das Protein aus der Zelle oder dem Zellüberstand isoliert. Stringente Hybridisierungsbedingungen sind vorzugsweise definiert gemäß Sambrook et al. (1989) Molecular Cloning A Laboratory Manual, Second Edition, Cold Spring Harbor Laboratory Press, und umfassen einen Waschschritt von 30 min in 0,1 x SSC, 0,5 % SDS bei 60°C und vorzugsweise 68°C.

Das erfindungsgemäße VLP kann weiterhin ein oder mehrere zusätzliche heterologe Proteine in der Kapsidstruktur aufweisen. Darunter ist zu verstehen, dass ein heterologes Protein in der Kapsidstruktur verankert ist, wobei bevorzugt mindestens ein Teil dieses Proteins von außen zugänglich ist. Als heterologes Protein eignen sich hierfür prinzipiell alle Proteine, die in die Kapsidstruktur inkorporiert werden können und die Selbstassemblierung des VLP nicht beeinträchtigen.

Das kationische Polymer, das mit dem VLP assoziiert wird, ist vorzugsweise ein physiologisch kompatibles Polymer. Beispiele für geeignete kationische Polymere sind Polyamine oder/und Polyimine, d.h. Polymere die primäre, sekundäre oder tertiäre Amino- oder /und Iminofunktionen in ausreichender Menge enthalten, um für eine positive Nettoladung des Polymers bei physiologischen Bedingungen zu sorgen. Günstigerweise beträgt das Verhältnis kationischer Gruppen zu anionischen Gruppen ≥ 2:1 besonders bevorzugt ≥ 5:1. Am meisten bevorzugt ist das kationische Polymer im Wesentlichen frei von anionischen Gruppen. Das Molekulargewicht der kationischen Polymere liegt vorzugsweise im Bereich von 10 bis 750 kD, besonders bevorzugt im Bereich von 25 bis 100 kD.

Spezifische Beispiele für kationische Polymere sind auf im Wesentlichen basischen Aminosäuren basierende Polymere, wie etwa Polylysin, insbesondere Poly-L-Lysin etc. Weitere spezifische Beispiele für geeignete kationische Polymere sind Polyalkylenimine, vorzugsweise Poly-C₂ - C₄ -Alkylenimine, insbesondere Polyethylenimin (PEI), pAMAM (Polyamidoamin)-Dendrimere und fraktionierte Dendrimere sowie kationisch modifiziertes Polyethylenglykol. Polyethylenimin ist ein besonders bevorzugtes kationisches Polymer im Sinne der vorliegenden Erfindung, da es nicht toxisch ist und eine hohe Dichte an positiven Ladungen aufweist. PEI ist weiterhin dazu in der Lage, nach Aufnahme in die Zellen eine pHabhängige Strukturveränderung zu bewirken, die zur Destabilisierung von endosomalen und lysosomalen Zellkompartimenten führt und somit die Freisetzung von Wirkstoffen, z.B. Nukleinsäuren, in das Zytoplasma erleichtert. Dieser Prozess wird durch die ausgeprägte Pufferkapazität der Iminogruppen unterstützt, die nach Azidifizierung in den Lysosomen protoniert werden und dann zu einer osmotischen Ruptur der Vesikelmembran führen.

Im Rahmen der vorliegenden Untersuchungen wurde nun festgestellt, das Polykationen eine hohe Affinität zu VP1-VLP aufweisen. Das bevorzugte Gewichtsverhältnis von VP1-VLP zu kationischem Polymer in den erfindungsgemäßen Konjugaten kann in breiten Bereichen variiert werden. So haben sich Gewichtsverhältnisse von 5:1 bis 1:10 als geeignet erwiesen, wobei Gewichtsverhältnisse von 2:1 und 1:5 besonders bevorzugt sind, um eine optimale Bindung zu ermöglichen.

In einer bevorzugten Ausführungsform der Erfindung ist mindestens ein Ligand an das kationische Polymer, das mit dem VP1-VLP assoziiert ist, gebunden. Der Ligand kann grundsätzlich eine beliebige Substanz sein, sofern er über kovalente oder/und nicht-kovalente Wechselwirkungen direkt oder indirekt an das kationische Polymer gebunden werden kann. Beispielsweise kann der Ligand eine zielzellspezifische Gruppe sein, z.B. ein Bindungspartner für einen Zelloberflächenrezeptor. Geeignete Beispiele für Bindungspartner sind natürliche Liganden oder synthetische Analoga davon, wobei hochmolekulare Liganden wie Proteine, z.B. Transferrin, Antikörper oder Zucker wie Mannose, aber auch niedermolekulare synthetische Liganden, z.B. das Tripeptid-Motiv R-G-D (Arg-Gly-Asp), eingesetzt werden können. Alternativ oder zusätzlich kann als Ligand auch eine Markierungsgruppe, z.B. eine durch geeignete Nachweismethoden erkennbare Gruppe, wie etwa eine Fluoreszenzmarkierungsgruppe oder Biotin, verwendet werden. Weiterhin kann der Ligand auch eine Effektorgruppe, z.B. eine cytotoxische Gruppe, sein. Selbstverständlich können auch Kombinationen mehrerer Liganden, insbesondere Kombinationen der zuvor genannten Liganden, eingesetzt werden.

In einer besonderen Ausführungsform kann das VLP im Inneren der Kapsidstruktur eine oder mehrere Wirksubstanzen enthalten. Unter Wirksubstanz wird in dieser Beschreibung jedes Molekül verstanden, welches in dem bei der Selbstassemblierung verwendeten Medium nicht üblicherweise vorhanden ist. Solche Wirksubstanzen umfassen z.B. Makromoleküle, wie etwa Nukleinsäuren, d.h. RNA, DNA oder artifizielle modifizierte Nukleinsäuren, sowie Proteine und andere physiologisch aktive Stoffe, die natürlicher, synthetischer oder rekombinanter Art sein können. Beispiele für derartige physiologisch aktive Stoffe sind z.B. Lipide, Phospholipide, Peptide, Arzneimittel, Toxine etc.

In einem anderen Aspekt betrifft die Erfindung ein Verfahren zur Herstellung von Konjugaten aus VP1-VLP und kationischen Polymeren, wobei mehrere VP1-Moleküle zu einem Partikel assemblieren und vor, während oder/und nach der Assemblierung ein kationisches Polymer zur Assoziierung an das Partikel zugesetzt wird. Vorzugsweise wird das kationische Polymer nach der Assemblierung zugesetzt. Besonders bevorzugt werden die VLP, insbesondere die rekombinanten VLP zuerst gereinigt, dann dissoziiert und anschließend in Gegenwart der Wirksubstanz reassoziiert. Im Falle, dass das Konjugat noch zusätzliche Wirksubstanzen enthalten soll, führt man die Assemblierung vorzugsweise in Gegenwart dieser weiteren Substanz durch, die dann im Inneren der Kapsidhülle des VLP eingeschlossen wird.

Bevorzugt wird das VLP auf rekombinante Weise hergestellt, wobei man eine für ein VP1-Protein kodierende Nukleinsäure in eine Zelle einbringt, die transformierte Zelle in einem Medium unter Bedingungen kultiviert, bei denen eine Expression der Nukleinsäure erfolgt, und das Expressionsprodukt aus der Zelle oder aus dem Medium gewinnt. Die Isolierung des rekombinanten VP1 erfolgt in Abhängigkeit des verwendeten Wirt/Vektorsystems direkt aus den Wirtszellen oder/und dem Zellkulturüberstand. Der Vorteil des rekombinanten Verfahrens liegt vor allem darin, dass auf einfache Weise VLP in hoher Reinheit und in großen Mengen gewonnen werden können. Als Expressionssystem hat sich in der Praxis die Verwendung von Baculoviren in Verbindung mit Insektenzellen, z.B. mit der Insektenzelllinie Sf 158, bewährt.

Zur Herstellung von VPL, die innerhalb der Kapsidstruktur ein heterologes Protein inkorporiert haben, bzw. von VPL, die im Inneren der Kapsidstruktur eine Wirksubstanz enthalten, modifiziert man das obige Herstellungsverfahren, indem man zu einem geeigneten Zeitpunkt, d.h, vor der Assemblierung der VLP die heterologen Proteine oder/und Wirksubstanzen in der gewünschten Menge bzw. Konzentration zusetzt und anschließend die Assemblierung zulässt. Auf diese Weise können VLP entstehen, welche heterologes Protein in der Kapsidhülle inkorporiert haben oder/und im Inneren eine eingeschlossene Wirksubstanz, z.B. eine Nukleinsäure enthalten. Die Inkorporation von heterologen Polypeptiden in der Kapsidhülle kann beispielsweise durch rekombinante Koexpression der jeweiligen Polypeptide, d.h. des VP1-Polypeptids und des heterologen Polypeptids in einer geeigneten Wirtszelle, z.B. einer eukaryontischen Zelle erfolgen. Die Inkorporation von Wirksubstanzen in das Innere der Kapsidhülle kann z.B. durch Dissoziation der Kapsidhülle und anschließende Reassoziation in Gegenwart der Wirksubstanz oder durch osmotischen Schock der VLP in Gegenwart der Wirksubstanz erfolgen.

Die erfindungsgemäßen Konjugate aus VLP und kationischen Polymer können für diagnostische und therapeutische Zwecke eingesetzt werden, z.B. zur Diagnose und Behandlung von Erkrankungen, die mit einer Infektion durch den JC-Virus assoziiert sind, wie etwa PML.

In einer weiteren Ausführungsform kann man die VLP als Transportvehikel verwenden, insbesondere zum Transport von Wirkstoffen zu einer Zielzelle und vorzugsweise in die Zielzelle. Durch Bindung eines Liganden an das mit dem VLP assoziierten kationischen Polymer wird dabei die Zielzellspezifität gegenüber unmodifizierten VLP signifikant verändert.

Auf diese Weise kann die Spezifität der Interaktion mit den vorgesehenen Zielzellen gewährleistet und in Abhängigkeit von der Anwendung auf eine Vielzahl an Zelltypen angepasst werden. Ein Beispiel für eine derartige Verwendung ist der zielgerichtete Transport von TNF-α Antisense-Nukleinsäuren an Oligodendrozyten bei Multipler Sklerose, da bekannt ist, dass bei dieser Erkrankung eine TNF-α Expression im Schub zur Demyelinisierung führt. Ein anderes Beispiel ist die Verwendung von VLP als Transportersystem für Nukleinsäuren in der Gentherapie.

Durch die Einschleusung des Thymidinkinase-Gens von Herpesviren wird der Selbstmordmechanismus der Zelle initiiert. Der zielgerichtete Transport des Tk-Gens (Thymidinkinase) in neoplastisch transformierte Zellen, wie z.B. Zellen der Benignen Prostata Hyperplasie, führt nach zusätzlicher Gabe eines Nukleosidanaloga (z.B. Acyclovir oder Gancyclovir) zur Termination der Replikation und in Folge zum Tod der transduzierten Zellen.

Die Erfindung wird nunmehr weiter erläutert durch die nachfolgenden Beispiele sowie die beigefügten Figuren und Sequenzprotokolle.

Es zeigen:

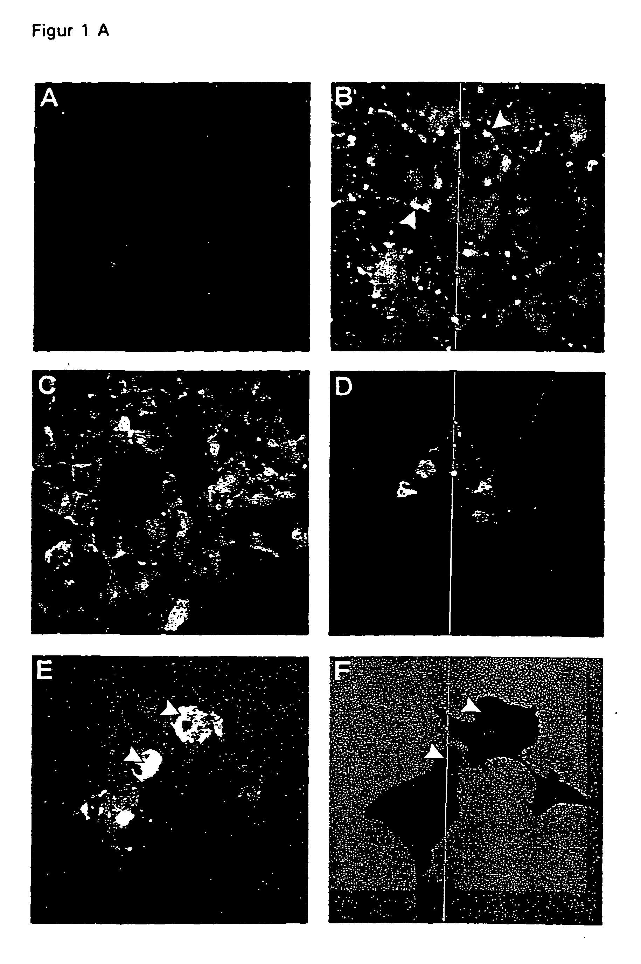

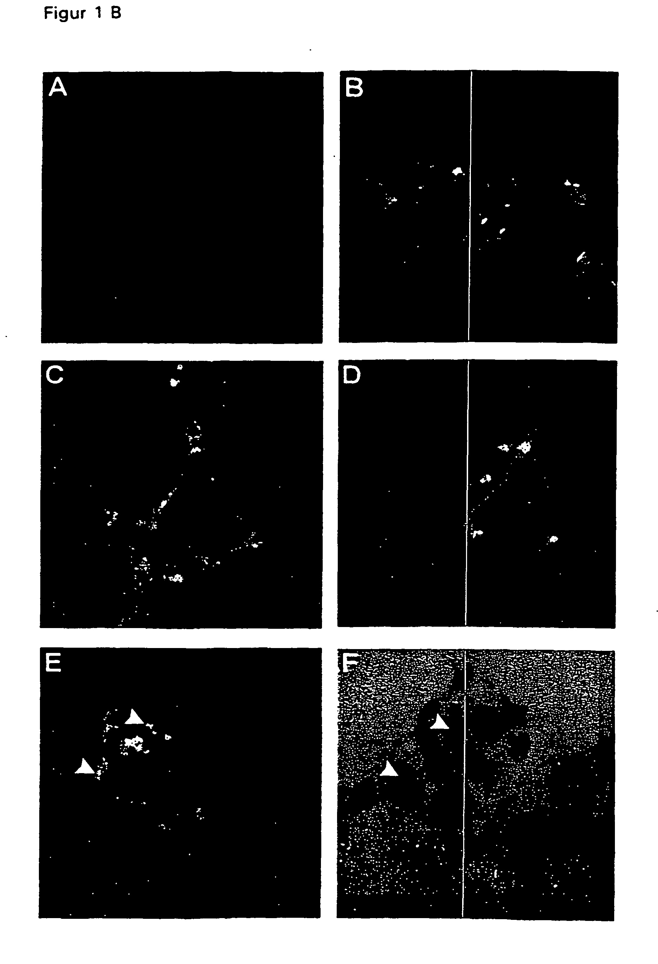

### Figur 1: VP1-spezifische Immunfluoreszenz zum Nachweis von VP1-VLP in SVG und COS-7 Zellen

30 µg VP1-VLP wurden mit jeweils 5 x 10⁴ Zellen für 24 Stunden inkubiert. Der Nachweis des VP1 erfolgte nach Fixierung der Zellen mit einem VP1-spezifischen Immunserum und einer anschließenden Inkubation mit einem FITC-gekoppelten anti-Kanninchen mAk durch fluoreszenzmikroskopische Methoden.

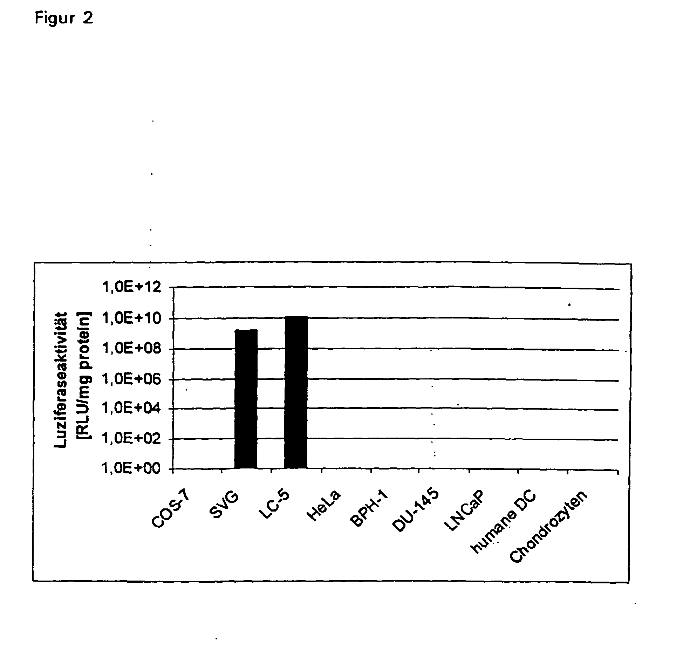

### Figur 2: Messung der Transduktionseffizienz von VP1-VLP in Zellen renalen und neuronalen Ursprungs

Zur Bestimmung der Transduktionseffizienz von VP1-VLP wurden in 1,25 µg VP1-VLP 1 µg DNA des Reporterplasmids pGL3-C verpackt. Jeweils 5 x 10⁴ Zellen wurden mit den VLP Komplexen für 24 Stunden inkubiert, anschließend gewaschen und für weitere 24 Stunden kultiviert. Im Anschluß daran wurde die Luziferase Aktivität in den Zelllysaten luminometrisch bestimmt.

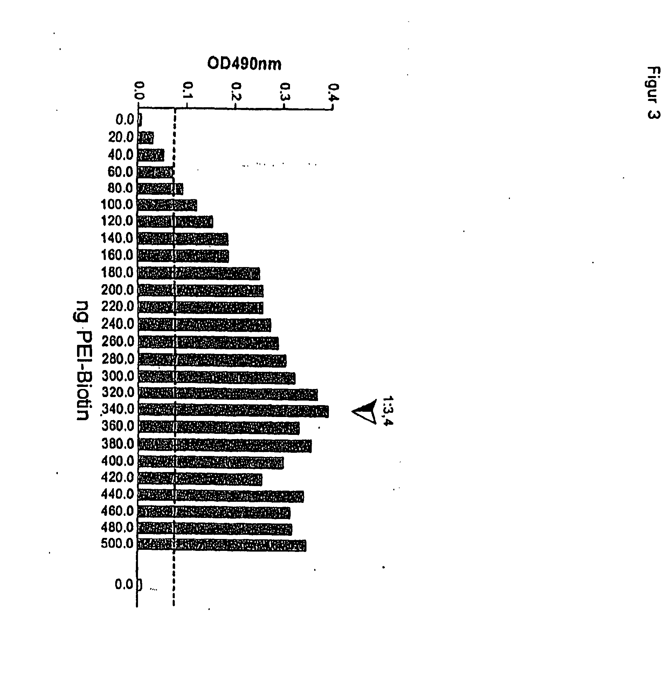

### Figur 3: Bestimmung der Bindungsfähigkeit von Polyethylenimin (PEI) an VP1-VLP

Zur Bestimmung der Bindungsfähigkeit von PEI an VP1-VLP wurde PEI-Biotin als detektierbares Konjugat verwendet. Dazu wurden in einem ELISA Test 100 µg VP1 pro Kavität gebunden, anschließend mit unterschiedlichen Konzentrationen PEI-Biotin inkubiert und die Komplexe mit Streptavidin HRP photometrisch bei 490 nm quantifiziert. Das VLP/PEI Verhältnis von 1:3,4 wurde als optimales Bindungsverhältnis ermittelt. Der Cut off-Wert der Messung ist durch die gestrichelte Linie gekennzeichnet.

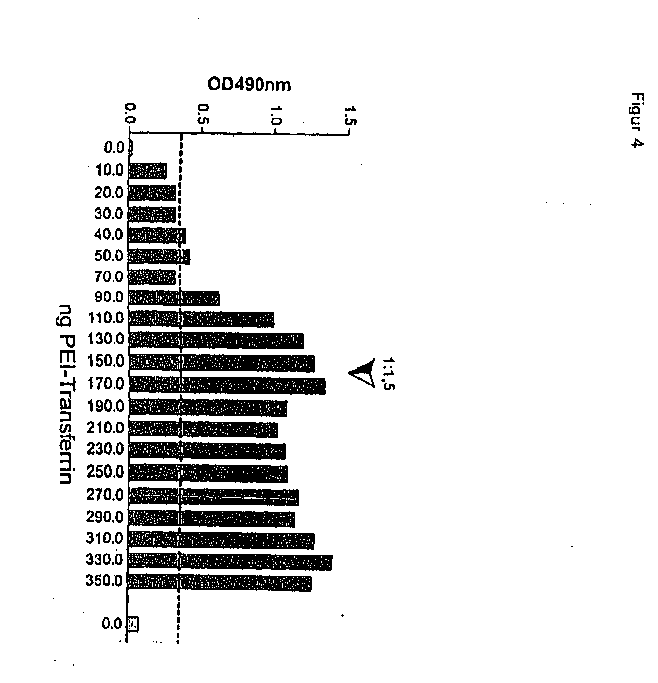

### Figur 4: Bestimmung der Bindungsfähigkeit von Polyethylenimin an VP1-VLP

Zur Bestimmung der Bindungsfähigkeit von PEI-Transferrin an VP1-VLP erfolgte in einem ELISA Test. Dazu wurden 100 µg VP1 pro Kavität gebunden, anschließend mit unterschiedlichen Konzentrationen PEI-Transferrin inkubiert und die Komplexe mit einem anti-Transferrin HRP konjugierten Antikörper photometrisch bei 490 nm quantifiziert. Das VLP/PEI Verhältnis von 1:1,5 wurde als optimales Bindungsverhältnis ermittelt. Der Cut off-Wert der Messung ist durch die gestrichelte Linie gekennzeichnet.

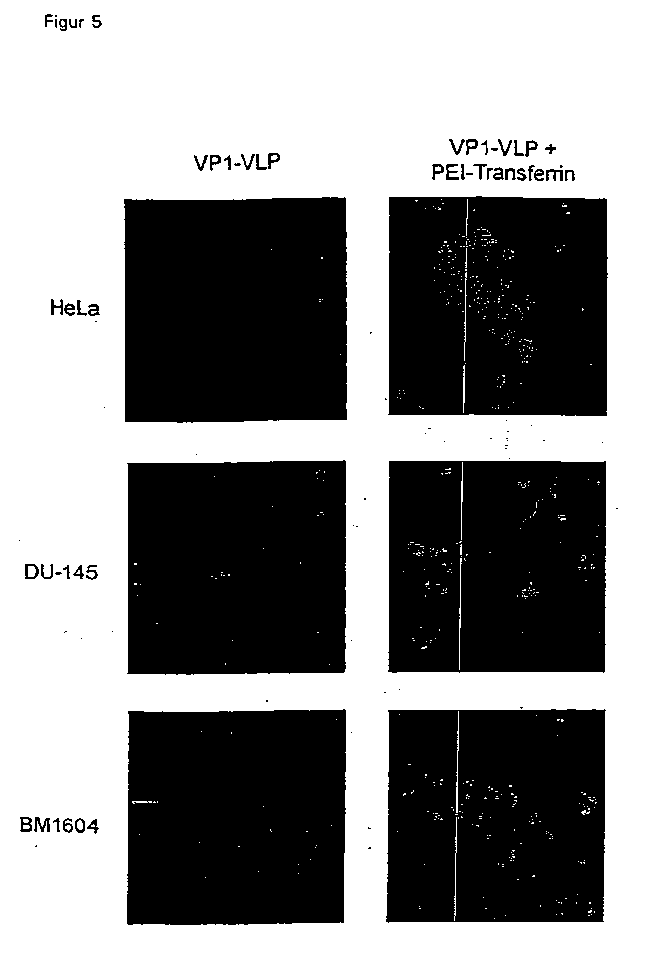

### Figur 5: VP1 spezifische Immunfluoreszenz zum Nachweis von VP1-VLP und VP1-VLP PEI/Transferrin Komplexen in HeLa, DU-145 und BM 1604 Zellen

3 *µ*g der VP1-VLP PEI/Transferrin Komplexe wurden mit jeweils 5x10⁴ Zellen für 24 Stunden inkubiert. Der Nachweis des VP1 erfolgte nach Fixierung der Zellen mit einem VP1 spezifischen Immunserum und einer anschließenden Inkubation mit einem FITC gekoppelten anti-Kaninchen mAk durch fluoreszenzmikroskopische Methoden.

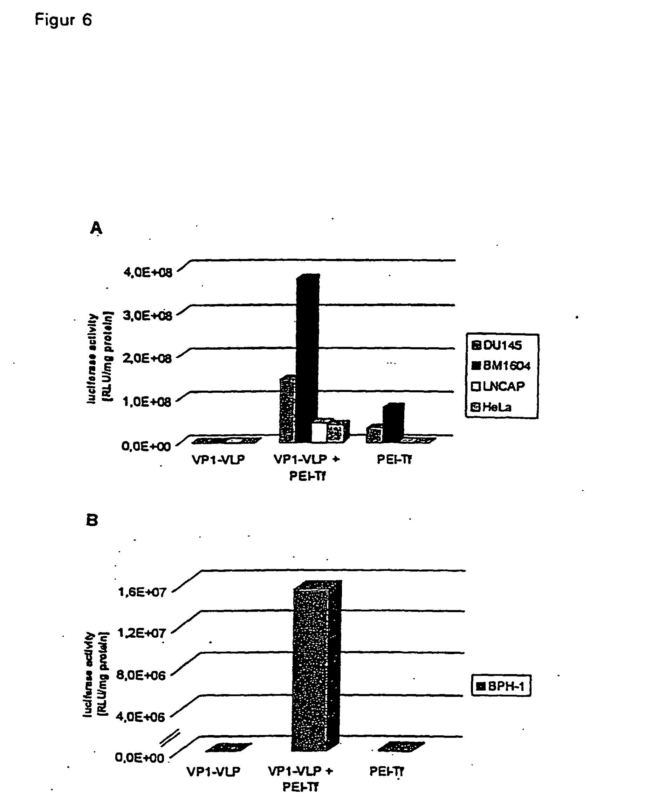

### Figur 6: Messung der Transduktionseffizienz von VP1-VLP/PEI-Transferrin Komplexen in verschiedenen Zelllinien

Zur Bestimmung der Transduktioneffizienz von VP1-VLP/PEI-Transferrin Komplexen wurden in 1,25 *µ*g VP1-VLP 1 *µ*g DNA des Reporterplasmids pGL3-C verpackt. Anschließend wurden die VLP mit 1,9 *µ*g PEI-Transferrin beladen. Jeweils 5x10⁴ Zellen wurden mit den VLP Komplexen für 24 Stunden inkubiert, anschließend gewaschen und für weitere 24 Stunden kultiviert. Im Anschluss daran wurde die Luziferase Aktivität in den Zelllysaten luminometrisch bestimmt. Als Kontrolle wurden neben den VLP Komplexen auch unbeladene VLP sowie DNA/PEI-Transferrin zur Transduktion der Zellen verwendet.

### Beispiele

### Beispiel 1: Spezifische Transduktion von Zellen neuronalen und renalen Ursprungs mittels JCV-VLP

Es wurde beobachtet, dass die JCV-VLP einen spezifischen Tropismus für Zellen renalen und neuronalen Ursprungs aufweisen. Für die Transduktionsversuche wurden die JCV-VLP aus dem Überstand einer Insektenzelllinie (SF158), die mit VP1-rekombinanten Bakuloviren infiziert waren, gereinigt. Für die Verpackung der DNA wurden 50 µg gereinigte VP1-VLP in einem Gesamtvolumen von 100 µl in 10 mM Tris-HCI, pH 7,5, 10 mM EGTA, 150 mM NaCI und 5 mM DTT dissoziiert. Um die DNA zu verpacken wurden VP1-Pentamere in Gegenwart der zu verpackenden Plasmide gegen einen Ca²⁺-haltigen Puffer dialysiert, um den Komplexbildner EGTA und das reduzierende Agens DTT zu verdünnen und gleichzeitig Ca²⁺-lonen zuzuführen. Die Bildung der JCV-VLP konnte elektronenmikroskopisch nachgewiesen werden. Um Plasmid-DNA zu entfernen, die an der Oberfläche der VP1-VLP gebunden war, wurde ein DNasel-Verdau durchgeführt. Je Reaktionsansatz wurden 10 U DNasel (Pharmacia Biotech, Freiburg) zugeführt, eine Endkonzentration von 10 mM Tris-HCI, pH 7,5 und 6 mM MgCl₂ eingestellt und eine Stunde bei 37°C inkubiert.

Mit diesen JCV-VLP konnten spezifisch Zellen neuronalen Ursprungs (wie z.B. SVG Zellen) und renalen Ursprungs (wie z.B. COS-7 Zellen) transduziert werden. Dazu wurden jeweils 5 x 10⁴ Zellen über Nacht bei 37°C mit den DNA-enthaltenden VP1-VLPs inkubiert. Die Transduktionseffizienz wurde mittels des Reportergens Luziferase bestimmt. In Zellen neuronalen und renalen Ursprungs wurden in diesen Versuchen hohe Transduktionseffizienzen gemessen. Dagegen wurde in Fribroblasten, T-Lymphozyten, Dentritischen Zellen, Chondrozyten, Zellen aus der Prostata oder aus Mammakarzinomen, die ebenfalls mit den JCV-VLP inkubiert wurden keine Luziferase Aktivitäten beobachtet.

### Beispiel 2: Charakterisierung der PEI-VLP Bindungseigenschaften

Da der Polymerisationsgrad des PEI und die Kopplungsdichte der Liganden Auswirkungen auf die Ladungsverteilung im Molekül hat, wurden zwei verschiedene PEI Präparationen (25 kD Polymer von SIGMA/Aldrich, Deisenhofen, Deutschland) in einem ELISA Test hinsichtlich ihrer Bindungseigenschaften an die VP1-VLP untersucht.

Dazu wurde zunächst Biotin als gut detektierbare Gruppe kovalent an Polyethylenimin in einem molaren Verhältnis von 1:1 gekoppelt. Die Bindungsfähigkeiten des PEI-Biotin an die VP1-VLPs wurde bestimmt, indem 100 ng VP1-VLP pro Kavität einer 96 well ELISA Platte gebunden wurde, dieses mit PEI-Biotin in verschiedenen Konzentrationen inkubiert und die Bindung mit einem Meerrettich-Peroxidase HPR) konjugierten Streptavidin Konjugat photometrisch quantifiziert wurde. Dabei ergab sich ein optimales VP1-VLP/PEI-Biotin Bindungsverhältnis (w/w) von 1:3,4 (Figur 3).

Analoge Versuche wurden mit dem PEI-Transferrin (Q-Biogene, Heidelberg, Deutschland) durchgeführt. Dazu wurden wiederum 100 ng VP1-VLPs pro Kavität auf einer ELISA Platte gebunden, mit verschiedenen Konzentrationen von PEI-Transferrin inkubiert und die Bindung anschließend mit einem monoklonalen anti-Transferrin Antikörper quantitativ nachgewiesen. Dabei ergab sich ein optimales VP1-VLP/PEI-Transferrin Bindungsverhältnis (w/w) von 1:1,5 (Figur 4).

### Beispiel 3: Zellbindungstest mit JCV VP1-VLP PEI-Transferrin

In Zellbindungstests wurde die Bindung, die Internalisierung und der Kerntransport der VP1-VLP/PEI-Transferrin Komplexe nachgewiesen. Die Beladung der VP1-VLP erfolgt in einem einfachen in vitro System. Dazu wurde ein VP1-VLP/PEI-Transferrin Gewichtsverhältnis von 1:1,5 eingestellt. Die Bindung erfolgte während einer 30 minütigen Inkubation bei 37°C. Die Zellbindungstests wurden an den Zelllinien HeLa (Scherer et al., J. Exp. Medicine 97 (1953), 695), DU145, BM1604 und BPH-1 (Mitchell et al., BJU International 85 (2000), 932) durchgeführt, die den Transferrin-Rezeptor exprimieren. Dazu wurden jeweils 5x10⁴ Zellen mit 3 µg VP1-VLP PEI/Transferrin für 24 Stunden inkubiert. Danach wurden die Zellen fixiert und das VP1 Protein mittels Immunfluoreszenz nachgewiesen (Figur 5). Bei allen vier Zelllinien war nach der Inkubation eine deutliche Bindung von VP1-VLP PEI/Transferrin an die Zellmembran zu erkennen. Bei den HeLa Zellen war bereits 2 Stunden nach Inkubation mit VP1-VLP PEI/Transferrin eine deutliche Anreicherung von VP1 an der Kernmembran erkennbar. In Kontrollexperimenten, die ohne vorherige PEI-Transferrin Behandlung durchgeführt wurden, konnte bei keiner der untersuchten Zelllinien eine Bindung von VP1-VLP an die Zellmembran beobachtet werden.

### Beispiel 4: Transduktion verschiedener Zellen mit VP1-VLP PEI-Transferrin

Die Transduktionseffizienz von VP1-VLP PEI/Transferrin wurde durch den Expressionsnachweis des Reportergens Luziferase bestimmt. Dazu wurde 1 µg des Plasmids pGL3-C (Promega) in 1,25 µg VP1-VLP verpackt. Anschließend wurden die VP1-VLPs mit PEI-Transferrin , wie in Beispiel 2 beschrieben, beladen. Die Inkubation der VP1-VLP PEI-Transferrin Komplexe mit den Zelllinien HeLa, DU145,BM1604 und BPH-1 erfolgte wie in Beispiel 3 beschrieben für 24 Stunden. Danach wurde das Medium gewechselt und die Zellen für weitere 24 Stunden kultiviert. Der quantitative Nachweis der Luziferase Expression erfolgte luminometrisch mittels eines kommerziell erhältlichen Systems (Promega).

Die Ergebnisse der Luziferase Messung demonstrieren eindrucksvoll, dass die Zellen die mittels unmodifizierter VP1-VLP nicht transduzierbar sind, nach vorheriger Beladung der VLP mit PEI-Transferrin effizient transduziert werden (Figur 6). Darüber hinaus zeigen die Luziferase Aktivitätsdaten, dass die Transduktion mit VP1-VLP/PEI-Transferrin Komplexen effizienter ist als die Verwendung herkömmlicher gebundener DNA an PEI-Transferrin. In diesen Untersuchungen konnte eindeutig gezeigt werden, dass kationische Polymere zur Verankerung von Liganden auf den VP1-VLP verwendbar sind, der enge Zielzelltropismus der VP1-VLP mittels zellspezifischer Liganden verändert werden kann und dass die Transduktions- und Expressionseffizienz gegenüber herkömmlich verwendetem PEI deutlich erhöht ist.

## Patentansprüche

1. Virus-ähnliches Partikel, das aus mehreren Molekülen des Virusprotein VP1 von JC-Virus aufgebaut ist, assoziiert mit einem kationischen Polymer.

2. Partikel nach Anspruch 1,
**dadurch gekennzeichnet,**
**dass** es aus rekombinantem VP1 aufgebaut ist.

3. Partikel nach Anspruch 1 oder 2,
**dadurch gekennzeichnet,**
**dass** das VP1 kodiert ist von einer Nukleinsäure, welche
(a) die in SEQ. ID NO. 1 gezeigte Nukleotidsequenz,
(b) eine der Sequenz aus (a) im Rahmen der Degeneration des genetischen Codes entsprechende Nukleotidsequenz oder/und
(c) eine mit einer zu den Sequenzen aus (a) oder/und (b) komplementären Sequenzen unter stringenten Bedingungen hybridisierende Nukleotidsequenz umfasst.

4. Partikel nach einem der Ansprüche 1 bis 3,
**dadurch gekennzeichnet,**
**dass** das kationische Polymer ein Polyamin oder Polyimin ist.

5. Partikel nach einem der Ansprüche 1 bis 4,
**dadurch gekennzeichnet,**
**dass** das kationische Polymer ein auf Aminosäuren basierendes Polymer, insbesondere ein Polylysin ist.

6. Partikel nach einem der Ansprüche 1 bis 4,
**dadurch gekennzeichnet,**
**dass** das kationische Polymer ein Polyalkylenimin, insbesondere ein Polyethylenimin (PEI) ist.

7. Partikel nach einem der Ansprüche 1 bis 6,
**dadurch gekennzeichnet,**
**dass** mindestens ein Ligand an das kationische Polymer gebunden ist.

8. Partikel nach Anspruch 7,
**dadurch gekennzeichnet,**
**dass** der Ligand eine zielzellspezifische Gruppe, insbesondere einen Bindungspartner für einen Zelloberflächenrezeptor umfasst.

9. Partikel nach Anspruch 7,
**dadurch gekennzeichnet,**
**dass** der Ligand eine Markierungsgruppe umfasst.

10. Partikel nach Anspruch 7,
**dadurch gekennzeichnet,**
**dass** der Ligand eine Effektorgruppe umfasst.

11. Partikel nach einem der Ansprüche 1 bis 10,
**dadurch gekennzeichnet,**
**dass** es im Inneren der Kapsidstruktur mindestens eine Wirksubstanz enthält.

12. Partikel nach Anspruch 11,
**dadurch gekennzeichnet,**
**dass** die Wirksubstanz ausgewählt ist aus Nukleinsäuren, Proteinen und physiologisch aktiven Stoffen.

13. Partikel nach Anspruch 11 oder 12,
**dadurch gekennzeichnet,**
**dass** die Wirksubstanz durch einen Dissoziations/Reassoziationszyklus in die VP1-VLP verpackt worden ist.

14. Verfahren zur Herstellung von Partikeln nach einem der Ansprüche 1 bis 13,
**dadurch gekennzeichnet,**
**dass** man
(a) mehrere VP1 Moleküle zu einem Partikel assembliert und
(b) vor, während oder/und nach der Assemblierung ein kationisches Polymer zur Assoziierung an das Partikel zusetzt.

15. Verfahren nach Anspruch 14,
**dadurch gekennzeichnet,**
**dass** das kationische Polymer nach der Assemblierung zugesetzt wird.

16. Verfahren nach einem der Ansprüche 14 oder 15,
**dadurch gekennzeichnet,**
**dass** man die Assemblierung in Gegenwart einer weiteren Substanz durchführt, wobei die Substanz im Inneren der Kapsidhülle eingeschlossen wird.

17. Diagnostisches Mittel, umfassend einen Partikel nach einem der Ansprüche 1 bis 13.

18. Therapeutisches Mittel, umfassend einen Partikel nach einem der Ansprüche 1 bis 13.

19. Verwendung eines Partikels nach einem der Ansprüche 1 bis 13 zur Herstellung eines Transportvehikels.

20. Verwendung eines Partikels nach einem der Ansprüche 1 bis 13 zur Herstellung eines Mittels zum Transport von Wirkstoffen zu einer Zielzelle, vorzugsweise in eine Zielzelle.

21. Verwendung nach Anspruch 20,
**dadurch gekennzeichnet,**
**dass** die Zielzelle neuronalen oder renalen Ursprungs ist.

22. Verwendung nach Anspruch 20 oder 21,
**dadurch gekennzeichnet,**
**dass** der Wirkstoff eine Nukleinsäure ist.

23. Verwendung eines Partikels nach einem der Ansprüche 1 bis 13 zur Herstellung eines Mittels zur spezifischen Transduktion von Zellen.

24. Verwendung nach Anspruch 23,
**dadurch gekennzeichnet,**
**dass** die Zelle neuronalen oder renalen Ursprungs ist.

25. Verwendung nach Anspruch 23 oder 24 zur Herstellung eines Mittels zur Gentherapie.

## Claims

1. Virus-like particle which is composed of several molecules of the viral protein VP1 derived from JC virus, which particle is associated with a cationic polymer.

2. Particle according to Claim 1,
**characterized in that**
it is composed of recombinant VP1.

3. Particle according to Claim 1 or 2,
**characterized in that**
the VP1 is encoded by a nucleic acid which comprises
(a) the nucleotide sequence shown in SEQ. ID NO. 1
(b) a nucleotide sequence which corresponds to the sequence from (a) within the context of the degeneracy of the genetic code, or/and
(c) a nucleotide sequence which hybridizes under stringent conditions to sequences complementary to one of the sequences from
(a) and/or (b).

4. Particle according to one of Claims 1 to 3,
**characterized in that**
the cationic polymer is a polyamine or polyimine.

5. Particle according to one of Claims 1 to 4,
**characterized in that**
the cationic polymer is a polymer based on amino acids, in particular a polylysine.

6. Particle according to one of Claims 1 to 4,
**characterized in that**
the cationic polymer is a polyalkylenimine, in particular a polyethylenimine (PEI).

7. Particle according to one of Claims 1 to 6,
**characterized in that**
at least one ligand is bound to the cationic polymer.

8. Particle according to Claim 7,
**characterized in that**
the ligand comprises a target cell-specific group, in particular a binding partner for a cell surface receptor.

9. Particle according to Claim 7,
**characterized in that**
the ligand comprises a labelling group.

10. Particle according to Claim 7,
**characterized in that**
the ligand comprises an effector group.

11. Particle according to one of Claims 1 to 10,
**characterized in that**
it contains at least one active substance within the capsid structure.

12. Particle according to Claim 11,
**characterized in that**
the active substance is selected from nucleic acids, proteins and physiologically active substances.

13. Particle according to Claim 11 or 12,
**characterized in that**
the active substance has been packaged into the VP1-VLPs by means of a dissociation/reassociation cycle.

14. Process for preparing particles according to one of Claims 1 to 13,
**characterized in that**
(a) several VP1 molecules are assembled to form a particle, and
(b) a cationic polymer is added, for association with the particle, before, during or/and after the assembly.

15. Process according to Claim 14,
**characterized in that**
the cationic polymer is added after the assembly.

16. Process according to one of Claims 14 or 15,
**characterized in that**
the assembly is carried out in the presence of an additional substance, with the substance being enclosed within the capsid coat.

17. Diagnostic agent comprising a particle according to one of Claims 1 to 13.

18. Therapeutic agent comprising a particle according to one of Claims 1 to 13.

19. Use of a particle according to one of Claims 1 to 13 for the manufacture of a transport vehicle.

20. Use of a particle according to one of Claims 1 to 13 for the manufacture of an agent for transporting active substances to a target cell, preferably into a target cell.

21. Use according to Claim 20,
**characterized in that**
the target cell is of neuronal or renal origin.

22. Use according to Claim 20 or 21,
**characterized in that**
the active compound is a nucleic acid.

23. Use of a particle according to one of Claims 1 to 13 for the manufacture of an agent for specifically transducing cells.

24. Use according to Claim 23,
**characterized in that**
the cell is of neuronal or renal origin.

25. Use according to Claim 23 or 24 for the manufacture of an agent for gene therapy.

## Revendications

1. Particule analogue à un virus, qui est constituée par plusieurs molécules de la protéine virale VP1 du virus JC, associée avec un polymère cationique.

2. Particule selon la revendication 1 **caractérisée en ce qu'**elle est constituée par VP1 recombinante.

3. Particule selon la revendication 1 ou 2 **caractérisée en ce que** VP1 est codée par un acide nucléique qui comprend
(a) la séquence nucléotidique montrée dans SEQ. ID NO. 1,
(b) une séquence nucléotidique correspondant à la séquence de (a) dans le cadre de la dégénérescence du code génétique et/ou
(c) une séquence nucléotidique qui s'hybride dans des conditions stringentes avec une séquence complémentaire des séquences de (a) et/ou (b).

4. Particule selon l'une des revendications 1 à 3 **caractérisée en ce que** le polymère cationique est une polyamine ou une polyimine.

5. Particule selon l'une des revendications 1 à 4 **caractérisée en ce que** le polymère cationique est un polymère à base d'aminoacides, en particulier une polylysine.

6. Particule selon l'une des revendications 1 à 4 **caractérisée en ce que** le polymère cationique est une polyalkylèneimine, en particulier une polyéthylèneimine (PEI).

7. Particule selon l'une des revendications 1 à 6 **caractérisée en ce qu'**au moins un ligand est lié au polymère cationique.

8. Particule selon la revendication 7 **caractérisée en ce que** le ligand comprend un groupe spécifique de cellules cible, en particulier un partenaire de liaison pour un récepteur de la surface cellulaire.

9. Particule selon la revendication 7 **caractérisée en ce que** le ligand comprend un groupe de marquage.

10. Particule selon la revendication 7 **caractérisée en ce que** le ligand comprend un groupe effecteur.

11. Particule selon l'une des revendications 1 à 10 **caractérisée en ce qu'**elle contient au moins une substance active à l'intérieur de la structure de capside.

12. Particule selon la revendication 11 **caractérisée en ce que** la substance active est choisie parmi les acides nucléiques, les protéines et les substances physiologiquement actives.

13. Particule selon la revendication 11 ou 12 **caractérisée en ce que** la substance active a été empaquetée dans la VP1-VLP par un cycle de dissociation/réassociation.

14. Procédé de production de particules selon l'une des revendications 1 à 13 **caractérisé en ce que**
(a) l'on assemble plusieurs molécules de VP1 en une particule et
(b) avant, pendant et/ou après l'assemblage on ajoute un polymère cationique pour l'association avec la particule.

15. Procédé selon la revendication 14 **caractérisé en ce que** le polymère cationique est ajouté après l'assemblage.

16. Procédé selon l'une des revendications 14 ou 15 **caractérisé en ce que** l'on réalise l'assemblage en présence d'une autre substance, la substance étant incluse à l'intérieur de l'enveloppe de capside.

17. Agent diagnostique comprenant une particule selon l'une des revendications 1 à 13.

18. Agent thérapeutique comprenant une particule selon l'une des revendications 1 à 13.

19. Utilisation d'une particule selon l'une des revendications 1 à 13 pour la production d'un véhicule de transport.

20. Utilisation d'une particule selon l'une des revendications 1 à 13 pour la production d'un agent pour le transport de principes actifs jusqu'à une cellule cible, de préférence dans une cellule cible.

21. Utilisation selon la revendication 20 **caractérisée en ce que** la cellule cible est d'origine neuronale ou rénale.

22. Utilisation selon la revendication 20 ou 21 **caractérisée en ce que** le principe actif est un acide nucléique.

23. Utilisation d'une particule selon l'une des revendications 1 à 13 pour la production d'un agent pour la transduction spécifique de cellules.

24. Utilisation selon la revendication 23 **caractérisée en ce que** la cellule est d'origine neuronale ou rénale.

25. Utilisation selon la revendication 23 ou 24 pour la production d'un agent pour la thérapie génique.


### SEQUENZPROTOKOLL

<110> Jenapharm GmbH & Co. KG
<120> Zusammensetzung zum zellspezifischen Transfer von Wirkstoffen
<130> 24684PEP DR deut.
<140>
   <141>
<150> DE 101 31 145.1-41
   <151> 2001-06-28
<160> 2
<170> Patientin Ver. 2.1
<210> 1
   <211> 1121
   <212> DNA
   <213> JCV
<220>
   <221> CDS
   <222> (39)..(1100)
<400> 1
   gtacgggact gcagcacctg ctcttgaagc atatgaag atg gcc cca aca aaa aga 56
                                             Met Ala Pro Thr Lys Arg
                                              1               5
   aaa gga gaa agg aag gac ccc gtg caa gtt cca aaa ctt ctt ata aga   104
   Lys Gly Glu Arg Lys Asp Pro Val Gln Val Pro Lys Leu Leu Ile Arg
               10                  15                  20
   gga gga gta gaa gtt cta gaa gtt aaa act ggg gtt gac tca att aca   152
   Gly Gly Val Glu Val Leu Glu Val Lys Thr Gly Val Asp Ser Ile Thr
           25                  30                  35
   gag gta gaa tgc ttt tta act cca gaa atg ggt gac cca gat gag cat   200
   Glu Val Glu Cys Phe Leu Thr Pro Glu Met Gly Asp Pro Asp Glu His
       40                  45                  50
   ctt agg ggt ttt agt aag tca ata tct ata tca gat aca ttt gaa agt   248
   Leu Arg Gly Phe Ser Lys Ser Ile Ser Ile Ser Asp Thr Phe Glu Ser
    55                  60                  65                  70
   gac tcc cca aat agg gac atg ctt cct tgt tac agt gtg gcc aga att    296
   Asp Ser Pro Asn Arg Asp Met Leu Pro Cys Tyr Ser Val Ala Arg Ile
                    75                  80                  85
   cca cta ccc aat cta aat gag gat cta acc tgt gga aat ata ctc atg    344
   Pro Leu Pro Asn Leu Asn Glu Asp Leu Thr Cys Gly Asn Ile Leu Met
                90                  95                 100
   tgg gag gct gtg acc tta aaa act gag gtt ata ggg gtg aca agt ttg    392
   Trp Glu Ala Val Thr Leu Lys Thr Glu Val Ile Gly Val Thr Ser Leu
           105                 110                 115
   atg aat gtg cac tct aat ggg caa gca act cat gac aat ggt gca ggg    440
   Met Asn Val His Ser Asn Gly Gln Ala Thr His Asp Asn Gly Ala Gly
       120                 125                 130
   aag cca gtg cag ggc acc agc ttt cat ttt ttt tct gtt ggg ggg gag    488
   Lys Pro Val Gln Gly Thr Ser Phe His Phe Phe Ser Val Gly Gly Glu
   135                 140                 145                 150
   gct tta gaa tta cag ggg gtg ctt ttt aat tac aga aca aag tac cca    536
   Ala Leu Glu Leu Gln Gly Val Leu Phe Asn Tyr Arg Thr Lys Tyr Pro
                   155                 160                 165
   gat gga aca att ttt cca aag aat gcc aca gtg caa tct caa gtc atg    584
   Asp Gly Thr Ile Phe Pro Lys Asn Ala Thr Val Gln Ser Gln Val Met
               170                 175                 180
   aac aca gag cac aag gcg tac cta gat aag aac aaa gca tat cct gtt    632
   Asn Thr Glu His Lys Ala Tyr Leu Asp Lys Asn Lys Ala Tyr Pro Val
           185                 190                 195
   gaa tgt tgg gtt cct gat ccc acc aga aat gaa aac aca aga tat ttt    680
   Glu Cys Trp Val Pro Asp Pro Thr Arg Asn Glu Asn Thr Arg Tyr Phe
       200                 205                 210
   ggg aca cta aca gga gga gaa aat gtt cct cca gtt ctt cat ata aca    728
   Gly Thr Leu Thr Gly Gly Glu Asn Val Pro Pro Val Leu His Ile Thr
   215                 220                 225                 230
   aac act gcc aca aca gtg ttg ctt gat gaa ttt ggt gtt ggg cca ctt    776
   Asn Thr Ala Thr Thr Val Leu Leu Asp Glu Phe Gly Val Gly Pro Leu
                   235                 240                 245
   tgc aaa ggt gac aac tta tac ttg tca gct gtt gat gtc tgt ggc atg    824
   Cys Lys Gly Asp Asn Leu Tyr Leu Ser Ala Val Asp Val Cys Gly Met
               250                 255                 260
   ttt aca aac agg tct ggt tcc cag cag tgg aga gga ctc tcc aga tat    872
   Phe Thr Asn Arg Ser Gly Ser Gln Gln Trp Arg Gly Leu Ser Arg Tyr
           265                 270                 275
   ttt aag gtg cag cta agg aaa agg agg gtt aaa aac ccc tac cca att    920
   Phe Lys Val Gln Leu Arg Lys Arg Arg Val Lys Asn Pro Tyr Pro Ile
       280                 285                 290
   tct ttc ctt ctt act gat tta att aac aga agg act cct aga gtt gat    968
   Ser Phe Leu Leu Thr Asp Leu Ile Asn Arg Arg Thr Pro Arg Val Asp
   295                 300                 305                 310
   ggg cag cct atg tat ggc atg gat gct caa gta gag gag gtt aga gtt    1016
   Gly Gln Pro Met Tyr Gly Met Asp Ala Gln Val Glu Glu Val Arg Val
                   315                 320                 325
   ttt gag gga aca gag gag ctt cca ggg gac cca gac atg atg aga tac    1064
   Phe Glu Gly Thr Glu Glu Leu Pro Gly Asp Pro Asp Met Met Arg Tyr
               330                 335                 340
   gtt gac aaa tat gga cag ttg cag aca aaa atg ctg taatcaaaag         1110
   Val Asp Lys Tyr Gly Gln Leu Gln Thr Lys Met Leu
           345                 350
   cttttattgt a                                                       1121
<210> 2
   <211> 354
   <212> PRT
   <213> JCV
<400> 2
   Met Ala Pro Thr Lys Arg Lys Gly Glu Arg Lys Asp Pro Val Gln Val
     1               5                  10                  15
   Pro Lys Leu Leu Ile Arg Gly Gly Val Glu Val Leu Glu Val Lys Thr
               20                  25                  30
   Gly Val Asp Ser Ile Thr Glu Val Glu Cys Phe Leu Thr Pro Glu Met
           35                  40                  45
   Gly Asp Pro Asp Glu His Leu Arg Gly Phe Ser Lys Ser Ile Ser Ile
       50                  55                  60
   Ser Asp Thr Phe Glu Ser Asp Ser Pro Asn Arg Asp Met Leu Pro Cys
   65                  70                  75                  80
   Tyr Ser Val Ala Arg Ile Pro Leu Pro Asn Leu Asn Glu Asp Leu Thr
   85 90 95
   Cys Gly Asn Ile Leu Met Trp Glu Ala Val Thr Leu Lys Thr Glu Val
   100 105 110
   Ile Gly Val Thr Ser Leu Met Asn Val His Ser Asn Gly Gln Ala Thr
   115 120 125
   His Asp Asn Gly Ala Gly Lys Pro Val Gln Gly Thr Ser Phe His Phe
   130 135 140
   Phe Ser Val Gly Gly Glu Ala Leu Glu Leu Gln Gly Val Leu Phe Asn
   145 150 155 160
   Tyr Arg Thr Lys Tyr Pro Asp Gly Thr Ile Phe Pro Lys Asn Ala Thr
   165 170 175
   Val Gln Ser Gln Val Met Asn Thr Glu His Lys Ala Tyr Leu Asp Lys
   180 185 190
   Asn Lys Ala Tyr Pro Val Glu Cys Trp Val Pro Asp Pro Thr Arg Asn
   195 200 205
   Glu Asn Thr Arg Tyr Phe Gly Thr Leu Thr Gly Gly Glu Asn Val Pro
   210 215 220
   Pro Val Leu His Ile Thr Asn Thr Ala Thr Thr Val Leu Leu Asp Glu
   225 230 235 240
   Phe Gly Val Gly Pro Leu Cys Lys Gly Asp Asn Leu Tyr Leu Ser Ala
   245 250 255
   Val Asp Val Cys Gly Met Phe Thr Asn Arg Ser Gly Ser Gln Gln Trp
   260 265 270
   Arg Gly Leu Ser Arg Tyr Phe Lys Val Gln Leu Arg Lys Arg Arg Val
   275 280 285
   Lys Asn Pro Tyr Pro Ile Ser Phe Leu Leu Thr Asp Leu Ile Asn Arg
   290 295 300
   Arg Thr Pro Arg Val Asp Gly Gln Pro Met Tyr Gly Met Asp Ala Gln
   305 310 315 320
   Val Glu Glu Val Arg Val Phe Glu Gly Thr Glu Glu Leu Pro Gly Asp
   325 330 335
   Pro Asp Met Met Arg Tyr Val Asp Lys Tyr Gly Gln Leu Gln Thr Lys
   340 345 350
   Met Leu

### SEQUENZPROTOKOLL

<110> Jenapharm GmbH & Co. KG
<120> Zusammensetzung zum zellspezifischen Transfer von Wirkstoffen
<130> 24684PEP_DR_deut.
<140>
   <141>
<150> DE 101 31 145.1-41
   <151> 2001-06-28
<160> 2
<170> PatentIn Ver. 2.1
<210> 1
   <211> 1121
   <212> DNA
   <213> JCV
<220>
   <221> CDS
   <222> (39)..(1100)
<400> 1
   gtacgggact gcagcacctg ctcttgaagc atatgaag atg gcc cca aca aaa aga 56
                                             Met Ala Pro Thr Lys Arg
                                               1               5
   aaa gga gaa agg aag gac ccc gtg caa gtt cca aaa ctt ctt ata aga   104
   Lys Gly Glu Arg Lys Asp Pro Val Gln Val Pro Lys Leu Leu Ile Arg
                10                  15                  20
   gga gga gta gaa gtt cta gaa gtt aaa act ggg gtt gac tca att aca   152
   Gly Gly Val Glu Val Leu Glu Val Lys Thr Gly Val Asp Ser Ile Thr
            25                  30                  35
   gag gta gaa tgc ttt tta act cca gaa atg ggt gac cca gat gag cat   200
   Glu Val Glu Cys Phe Leu Thr Pro Glu Met Gly Asp Pro Asp Glu His
        40                  45                  50
   ctt agg ggt ttt agt aag tca ata tct ata tca gat aca ttt gaa agt   248
   Leu Arg Gly Phe Ser Lys Ser Ile Ser Ile Ser Asp Thr Phe Glu Ser
    55                  60                  65                  70
   gac tcc cca aat agg gac atg ctt cct tgt tac agt gtg gcc aga att    296
   Asp Ser Pro Asn Arg Asp Met Leu Pro Cys Tyr Ser Val Ala Arg Ile
                   75                  80                  85
   cca cta ccc aat cta aat gag gat cta acc tgt gga aat ata ctc atg    344
   Pro Leu Pro Asn Leu Asn Glu Asp Leu Thr Cys Gly Asn Ile Leu Met
               90                  95                  100
   tgg gag gct gtg acc tta aaa act gag gtt ata ggg gtg aca agt ttg    392
   Trp Glu Ala Val Thr Leu Lys Thr Glu Val Ile Gly Val Thr Ser Leu
           105                 110                 115
   atg aat gtg cac tct aat ggg caa gca act cat gac aat ggt gca ggg    440
   Met Asn Val His Ser Asn Gly Gln Ala Thr His Asp Asn Gly Ala Gly
       120                 125                 130
   aag cca gtg cag ggc acc agc ttt cat ttt ttt tct gtt ggg ggg gag    488
   Lys Pro Val Gln Gly Thr Ser Phe His Phe Phe Ser Val Gly Gly Glu
   135                 140                 145                 150
   gct tta gaa tta cag ggg gtg ctt ttt aat tac aga aca aag tac cca    536
   Ala Leu Glu Leu Gln Gly Val Leu Phe Asn Tyr Arg Thr Lys Tyr Pro
                   155                 160                 165
   gat gga aca att ttt cca aag aat gcc aca gtg caa tct caa gtc atg    584
   Asp Gly Thr Ile Phe Pro Lys Asn Ala Thr Val Gln Ser Gln Val Met
               170                 175                 180
   aac aca gag cac aag gcg tac cta gat aag aac aaa gca tat cct gtt    632
   Asn Thr Glu His Lys Ala Tyr Leu Asp Lys Asn Lys Ala Tyr Pro Val
           185                 190                 195
   gaa tgt tgg gtt cct gat ccc acc aga aat gaa aac aca aga tat ttt    680
   Glu Cys Trp Val Pro Asp Pro Thr Arg Asn Glu Asn Thr Arg Tyr Phe
       200                 205                 210
   ggg aca cta aca gga gga gaa aat gtt cct cca gtt ctt cat ata aca    728
   Gly Thr Leu Thr Gly Gly Glu Asn Val Pro Pro Val Leu His Ile Thr
   215                 220                 225                 230
   aac act gcc aca aca gtg ttg ctt gat gaa ttt ggt gtt ggg cca ctt    776
   Asn Thr Ala Thr Thr Val Leu Leu Asp Glu Phe Gly Val Gly Pro Leu
                   235                 240                 245
   tgc aaa ggt gac aac tta tac ttg tca gct gtt gat gtc tgt ggc atg    824
   Cys Lys Gly Asp Asn Leu Tyr Leu Ser Ala Val Asp Val Cys Gly Met
               250                 255                 260
   ttt aca aac agg tct ggt tcc cag cag tgg aga gga ctc tcc aga tat     872
   Phe Thr Asn Arg Ser Gly Ser Gln Gln Trp Arg Gly Leu Ser Arg Tyr
           265                 270                 275
   ttt aag gtg cag cta agg aaa agg agg gtt aaa aac ccc tac cca att     920
   Phe Lys Val Gln Leu Arg Lys Arg Arg Val Lys Asn Pro Tyr Pro Ile
       280                 285                 290
   tct ttc ctt ctt act gat tta att aac aga agg act cct aga gtt gat     968
   Ser Phe Leu Leu Thr Asp Leu Ile Asn Arg Arg Thr Pro Arg Val Asp
   295                 300                 305                 310
   ggg cag cct atg tat ggc atg gat gct caa gta gag gag gtt aga gtt     1016
   Gly Gln Pro Met Tyr Gly Met Asp Ala Gln Val Glu Glu Val Arg Val
                   315                 320                 325
   ttt gag gga aca gag gag ctt cca ggg gac cca gac atg atg aga tac     1064
   Phe Glu Gly Thr Glu Glu Leu Pro Gly Asp Pro Asp Met Met Arg Tyr
               330                 335                 340
   gtt gac aaa tat gga cag ttg cag aca aaa atg ctg taatcaaaag          1110
   Val Asp Lys Tyr Gly Gln Leu Gln Thr Lys Met Leu
           345                 350
   cttttattgt a                                                        1121
<210> 2
   <211> 354
   <212> PRT
   <213> JCV
<400> 2
   Met Ala Pro Thr Lys Arg Lys Gly Glu Arg Lys Asp Pro Val Gln Val
    1              5                   10                  15
   Pro Lys Leu Leu Ile Arg Gly Gly Val Glu Val Leu Glu Val Lys Thr
               20                  25                  30
   Gly Val Asp Ser Ile Thr Glu Val Glu Cys Phe Leu Thr Pro Glu Met
           35                  40                  45
   Gly Asp Pro Asp Glu His Leu Arg Gly Phe Ser Lys Ser Ile Ser Ile
       50                  55                  60
   Ser Asp Thr Phe Glu Ser Asp Ser Pro Asn Arg Asp Met Leu Pro Cys
   65                  70                  75                  80
   Tyr Ser Val Ala Arg Ile Pro Leu Pro Asn Leu Asn Glu Asp Leu Thr
                   85                  90                  95
   Cys Gly Asn Ile Leu Met Trp Glu Ala Val Thr Leu Lys Thr Glu Val
               100                 105                 110
   Ile Gly Val Thr Ser Leu Met Asn Val His Ser Asn Gly Gln Ala Thr
           115                 120                 125
   His Asp Asn Gly Ala Gly Lys Pro Val Gln Gly Thr Ser Phe His Phe
       130                 135                 140
   Phe Ser Val Gly Gly Glu Ala Leu Glu Leu Gln Gly Val Leu Phe Asn
   145                 150                 155                 160
   Tyr Arg Thr Lys Tyr Pro Asp Gly Thr Ile Phe Pro Lys Asn Ala Thr
                   165                 170                 175
   Val Gln Ser Gln Val Met Asn Thr Glu His Lys Ala Tyr Leu Asp Lys
               180                 185                 190
   Asn Lys Ala Tyr Pro Val Glu Cys Trp Val Pro Asp Pro Thr Arg Asn
           195                 200                 205
   Glu Asn Thr Arg Tyr Phe Gly Thr Leu Thr Gly Gly Glu Asn Val Pro
       210                 215                 220
   Pro Val Leu His Ile Thr Asn Thr Ala Thr Thr Val Leu Leu Asp Glu
   225                 230                 235                 240
   Phe Gly Val Gly Pro Leu Cys Lys Gly Asp Asn Leu Tyr Leu Ser Ala
                   245                 250                 255
   Val Asp Val Cys Gly Met Phe Thr Asn Arg Ser Gly Ser Gln Gln Trp
               260                 265                 270
   Arg Gly Leu Ser Arg Tyr Phe Lys Val Gln Leu Arg Lys Arg Arg Val
           275                 280                 285
   Lys Asn Pro Tyr Pro Ile Ser Phe Leu Leu Thr Asp Leu Ile Asn Arg
       290                 295                 300
   Arg Thr Pro Arg Val Asp Gly Gln Pro Met Tyr Gly Met Asp Ala Gln
   305                 310                 315                 320
   Val Glu Glu Val Arg Val Phe Glu Gly Thr Glu Glu Leu Pro Gly Asp
                   325                 330                 335
   Pro Asp Met Met Arg Tyr Val Asp Lys Tyr Gly Gln Leu Gln Thr Lys
               340                 345                 350
   Met Leu